(19) Europäisches Patentamt
European Patent Office
Office européen des brevets

(11) EP 1 273 564 B1

(12) **EUROPÄISCHE PATENTSCHRIFT**

(45) Veröffentlichungstag und Bekanntmachung des Hinweises auf die Patenterteilung:
**20.02.2008 Patentblatt 2008/08**

(51) Int Cl.:
*C07C 45/50* (2006.01) *B01J 23/46* (2006.01)

(21) Anmeldenummer: **02014211.3**

(22) Anmeldetag: **26.06.2002**

(54) **Verfahren zur Herstellung von Aldehyden**

Process for the preparation of aldehydes

Procédé pour la préparation d'aldéhydes

(84) Benannte Vertragsstaaten:
**AT BE CH CY DE DK ES FI FR GB GR IE IT LI LU MC NL PT SE TR**

(30) Priorität: **07.07.2001 DE 10133072**

(43) Veröffentlichungstag der Anmeldung:
**08.01.2003 Patentblatt 2003/02**

(73) Patentinhaber: **OXEA GmbH
46147 Oberhausen (DE)**

(72) Erfinder:
- **Bohnen, Hans, Dr.
47441 Moers (DE)**
- **Frohning, Carl Dieter, Dr.
46485 Wesel (DE)**
- **Wiebus, Ernst
46147 Oberhausen (DE)**

(56) Entgegenhaltungen:
**EP-A- 0 246 475** **EP-A- 0 544 091**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 273 564 B1

## Beschreibung

Die vorliegende Erfindung betrifft ein Verfahren zur Herstellung von Aldehyden in Gegenwart eines Katalysators auf der Basis von Rhodium, worin während des Verfahrens zusätzliches Rhodium in der Form eines spezifischen Umsetzungsproduktes dem Verfahren zugesetzt wird. Die Erfindung betrifft weiterhin die Verwendung des genannten Umsetzungsproduktes in einem Hydroformylierungsverfahren.

Es ist bekannt, durch übergangsmetallkatalysierte Umsetzungen von Olefinen mit Kohlenmonoxid und Wasserstoff (Hydroformylierung) Aldehyde und Alkohole herzustellen, die ein Kohlenstoffatom mehr als das Ausgangsolefin enthalten. Neben Kobalt, das als Katalysatormetall in großem Umfang technisch Anwendung findet, hat insbesondere Rhodium in den letzten Jahren zunehmend an Bedeutung gewonnen.

Bei den in der Technik eingeführten Verfahren ist der Rhodium-Katalysator im allgemeinen ein durch zusätzliche Liganden, insbesondere tertiäre organische Phosphine oder Phosphite modifiziertes Hydridorhodiumcarbonyl. Meist liegen die Liganden im Überschuß vor, so daß das Katalysatorsystem aus Komplexverbindungen und freiem Liganden besteht, wobei das Katalysatorsystem in dem organischen Reaktionsprodukt gelöst vorliegt. Der Einsatz der beschriebenen Rhodium-Katalysatoren ermöglicht es, die Hydroformylierungsreaktionen bei Drücken unter 30 MPa durchzuführen.

Die Hydroformylierungsreaktion wird dabei in der Regel nach dem Aldehydaustrag gefahren. Nach einer gewissen Betriebslaufzeit beobachtet man einen Aktivitätsabfall, der sich in einem verminderten Aldehydaustrag bemerkbar macht. Als Ursache für den Aktivitätsabfall vermutet man das vermehrte Auftreten von Katalysator- und Ligandenabbauprodukten, die mit dem Rhodium-Katalysator solche Komplexverbindungen eingehen, die nur noch geringe katalytische Aktivitäten aufweisen oder eine zunehmende Konzentration an Höhersiedern im Reaktionsgemisch.

Um diesem Aktivitätsabfall entgegenzuwirken, schlägt die EP-A-0544091 den Zusatz von Maleinsäureanhydrid, Fumarsäure oder anderen olefinisch ungesättigten Verbindungen zu einer gebrauchten Altkatalysatorlösung vor. Diese Verbindungen reagieren mit den schädlichen Abbauprodukten unter Bildung von Verbindungen, die die Aktivität des Rhodiumkatalysators offenbar nicht mehr beeinträchtigen. Nachteilig bei diesem Verfahren ist der recht hohe Maleinsäureanhydridzusatz. Dieses Verfahren hat sich daher bisher in der Technik nicht durchsetzen können.

Gegenstand der Deutschen Offenlegungsschrift DE-A1-199 40 249 ist ein Verfahren zur Herstellung von Aldehyden unter Verwendung einer wäßrigen Katalysatorlösung, die neben Rhodium und wasserlöslichen Phosphinen Salze aromatischer Sulfon-, Carbon- oder Phosphonigsäuren enthält. Der Zusatz dieser Salze zu gebrauchten Hydroformylierungs-Altkatalysatorlösungen führt zu einer deutlichen Aktivitätssteigerung in der Hydroformylierungsreaktion. Auch der Zusatz dieser Salze zu frisch hergestellten Katalysatorlösungen bewirkt einen stabilisierenden Effekt und verlängert die Katalysatorlebensdauer. Aber auch dieses Verfahren erfordert den Zusatz von zusätzlichen, salzartigen Stoffen zu der wäßrigen Katalysatorlösung, was zu einem schnelleren Überschreiten der Löslichkeitsgrenzen des Liganden bzw. dessen Abbauprodukten führt. Die Verkürzung des Lebenszyklus der Katalysatorlösung oder der Katalysatorstandzeit ist die Folge.

Die EP-A-0269964 beschreibt ein Verfahren zur Herstellung von Aldehyden, worin zur Aufrechterhaltung der ursprünglichen Phosphinkonzentration solange frische Phosphinlösung zugeführt wird, bis die Gesamtkonzentration an komplexbildenden Phosphinen und zur Komplexbildung nichtfähigen Folge- und Abbauprodukten der Phosphine etwa 35 bis 45 Gew.-%, bezogen auf die wäßrige Lösung, beträgt.

Bei der technischen Durchführung der Hydroformylierungsreaktion steuert man den Aktivitätsabfall der Katalysatorlösung bzw. den verminderten Aldehydaustrag in der Regel durch eine Temperaturerhöhung entgegen. Nachteilig bei dieser Maßnahme ist jedoch eine verstärkte thermische Belastung des Katalysatorsystems, die wiederum zu einer vermehrten Schädigung des Katalysators führt.

Die Erfinder fanden, dass sich der Aldehydaustrag prinzipiell auch erhöhen läßt, indem man anstelle einer Temperaturerhöhung frühzeitig mit der Ergänzung von frischen Rhodiumverbindungen, z.B. von Rhodiumacetylacetonat oder Rhodiumcarboxylaten beginnt.

Der Einsatz einer präformierten, d.h. einer durch die vorherige Umsetzung einer Rhodiumverbindung mit Kohlenmonoxid und Wasserstoff erhaltenen Form des Rhodiumkatalysators als Ausgangskatalysator für die Hydroformylierungsreaktion ist aus EP-A-0246475 bekannt. Dort dient der Einsatz des präformierten Katalysators zu Beginn der Hydroformylierungsreaktion dazu, die Reaktionszeit in der Anfangsphase der Umsetzung zu verkürzen und den Austrag von Edelmetall in diesem Reaktionsabschnitt zu unterbinden.

Auch die Deutsche Offenlegungsschrift DE-A1-199 40 249 weist auf die Möglichkeit hin, den Rhodium-Katalysator vor dem Einsatz als Startkatalysator in der Hydroformylierungsreaktion einer Vorbehandlung in Gegenwart von Kohlenmonoxid und Wasserstoff zu unterziehen.

In der EP-B1-0 695 734 ist ein Präformierungsschritt Bestandteil eines Verfahrens zur Rhodiumrückgewinnung aus dem Austrag der Hydroformylierung.

Der zuvor genannte Stand der Technik enthält jedoch keinen Hinweis darauf, den beim Hydroformylierungsverfahren stattfindenden Aktivitätsverlust durch die Zugabe von weiterem Rhodium zu verhindern, und folglich enthält

der genannte Stand der Technik auch keinen Hinweis darauf, in welcher Form weiteres Rhodium zur optimalen Verhinderung des Aktivitätsverlust zugesetzt werden kann.

Es wurde nun gefunden, dass sich die Produktivität der Hydroformylierungsreaktion unter Rhodiumergänzung deutlich verbessern läßt, wenn man eine Rhodiumverbindung nicht unbehandelt in den Hydroformylierungsreaktor gibt, sondern erst, nachdem sie einer Vorbehandlung mit Kohlenmonoxid und Wasserstoff in Gegenwart von organischen Phosphorverbindungen oder deren Salzen unterworfen wurde.

Dies ist insbesondere deshalb überraschend, weil die Vorbehandlung ja an sich unter den Bedingungen der Hydroformylierungsreaktion ($CO/H_2$ - Druck) stattfindet. Der Fachmann hätte daher erwartet, dass eine separate, vorherige Umsetzung des ergänzend zugesetzten Rhodiums bzw. der Rhodiumverbindung mit Kohlenmonoxid und Wasserstoff unter Druck überflüssig ist, weil eine solche Umsetzung ja auch im Hydroformylierungsreaktor unter ähnlichen Bedingungen stattfinden würde. Diese Erwartung war jedoch völlig überraschend unzutreffend. Offenbar wird unter den Bedingungen der laufenden Hydroformylierung ein Teil des ergänzend zugesetzten Rhodiums bzw. der Rhodiumverbindung nicht in die aktive Spezies überführt. Wird hingegen das Rhodium bzw. die Rhodiumverbindung vorher einem separaten Vorbehandlungsschritt in Gegenwart von rhodiumkomplexbildenden Verbindungen mit Kohlenmonoxid und Wasserstoff unterworfen, kann das dabei generierte Umsetzungsprodukt seine Aktivität in der laufenden Hydroformylierung gewissermaßen verlustfrei entfalten. Die vorliegende Erfindung vermag daher die Effizienz der Hydroformylierung in Bezug auf das eingesetzte Rhodium enorm zu steigern.

Ohne sich an diese Theorie zu binden, wird vermutet, dass eine derart vorbehandelte Rhodiumverbindung in einer solchen Form vorzuliegen scheint, in der sie mit Ligandenabbauprodukten des Rhodiumkatalysators der Hydroformylierungsreaktion keine Konkurrenzreaktion eingeht sondern ihre katalytische Aktivität vollständig entfalten kann.

Die Aufgabe der Erfindung bestand somit darin, ein Verfahren zur Herstellung von Aldehyden durch die Umsetzung olefinisch ungesättigter Verbindungen mit Kohlenmonoxid und Wasserstoff zu finden (im folgenden kurz Hydroformylierungsverfahren genannt), das auch über einen längeren Zeitraum mit hoher Aktivität durchgeführt werden kann, ohne dass eine Erhöhung der Reaktortemperatur, die mit einem verstärkten Katalysatorabbau einhergeht, oder der Zusatz großer Mengen fremder, die Aktivität des Katalysators steigernder, zusätzlicher Verbindungen erforderlich wären, und worin das eingesetzte Rhodium eine optimale Nutzung erfährt.

Gegenstand der Erfindung ist somit ein Verfahren zur Herstellung von Aldehyden durch Umsetzung olefinisch ungesättigter Verbindungen mit Kohlenmonoxid und Wasserstoff bei Temperaturen von 20 bis 200°C und Drücken von 0,1 bis 50 MPa in flüssiger Phase in Gegenwart eines Katalysators auf der Basis von Rhodium, worin während des Verfahrens zusätzliches Rhodium in der Form eines Umsetzungsproduktes zugesetzt wird, dessen Herstellung die Umsetzung von einem oder mehreren Rhodiumsalzen einer organischen Verbindung, ausgewählt aus der Gruppe von Rhodiumsalzen, die aus Rhodium(III)-salzen von Beta-Diketonen und Rhodiumsalzen von Carbonsäuren besteht, in Gegenwart von organischen Phosphorverbindungen oder deren Salzen mit Kohlenmonoxid und Wasserstoff umfaßt.

In dem Verfahren zur Herstellung von Aldehyden werden olefinisch ungesättigte Verbindungen, bevorzugt mit 3 bis 20 Kohlenstoffatomen, mit Kohlenmonoxid und Wasserstoff bei einer Temperatur von 20 bis 200°C, bevorzugt 40 bis 150°C und einem Druck von 0,1 bis 50 MPa, bevorzugt 1 bis 20 MPa in Gegenwart eines Katalysators auf der Basis von Rhodium umgesetzt. Weiterhin findet die Umsetzung bevorzugt in Gegenwart phosphorhaltiger Verbindungen statt, die im allgemeinen als Liganden im Katalysatorsystem fungieren.

Bei den organischen Phosphorverbindungen oder deren Salzen handelt es sich zweckmäßigerweise um solche Verbindungen, die auch in der Hydroformylierungsreaktion als Liganden eingesetzt werden. Daher haben sich besonders phosphorhaltige Verbindungen bewährt.

Die Bedingungen der Hydroformylierungsreaktion sind an sich bekannt, und es kann beispielsweise auf den in der Einleitung erwähnten Stand der Technik Bezug genommen werden.

Die olefinisch ungesättigte Verbindung kann eine oder mehr als eine Kohlenstoff-Kohlenstoff-Doppelbindung enthalten. Die Kohlenstoff-Kohlenstoff-Doppelbindung kann endständig oder innenständig (interne Olefine) angeordnet sein. Bevorzugt sind olefinische Verbindungen mit endständiger Kohlenstoff-Kohlenstoff-Doppelbindung.

Beispiele für $\alpha$-olefinische Verbindungen (mit endständiger Kohlenstoff-Kohlenstoff-Doppelbindung) sind Alkene, Alkylenalkanoate, Alkylenalkylether und Alkenole, insbesondere solche mit 3 bis 12 Kohlenstoffatomen. Ohne Anspruch auf Vollständigkeit seien als $\alpha$-olefinische Verbindungen Propylen, 1-Buten, 1-Penten, 1-Hexen, 1-Hepten, 1-Octen, 1-Decen, 1-Dodecen, 1-Octadecen, 2-Ethyl-1-hexen, Styrol, 3-Phenyl-1-propen, Allylchlorid, 1,4-Hexadien, 1,3-Butadien, 1,7-Octadien, 3-Cyclohexyl-1-buten, Hex-1-en-4-ol, Oct-1-en-4-ol, Vinylcyclohexen, n-Propyl-7-octenoat, 7-Octensäure, 5-Hexenamid genannt.

Als Beispiele weiterer geeigneter olefinischer Verbindungen seien Buten-2, Diisobutylen, Tripropylen, Raffinat II (Gemisch aus 1-Buten, 2-Buten und Butan), Octol oder Dimersol (Dimerisierungsprodukte von Butenen), Tetrapropylen, Cyclohexen, Dicyclopentadien, acyclische, cyclische oder bicyclische Terpene, wie Myrcen, Limonen und Pinen erwähnt. Die Hydroformylierung wird bevorzugt mit olefinischen Verbindungen mit 3 bis 12 Kohlenstoffatomen durchgeführt.

Die Herstellung der Aldehyde kann in flüssiger Phase einphasig in einer organischen Phase (Homogenverfahren) oder in flüssiger Phase zweiphasig in Gegenwart einer organischen und einer wässrigen Phase (Heterogenverfahren) erfolgen. Bevorzugt findet die Umsetzung der Olefine nach dem Heterogenverfahren in Gegenwart einer organischen und einer wässrigen Phase statt.

Die Herstellung der Aldehyde erfolgt im allgemeinen in Gegenwart von organischen Phosphorverbindungen oder deren Salzen.

Die Wahl der organischen Phosphorverbindungen richtet sich zweckmäßig nach der Art des Hydroformylierungsverfahren. Wird die Hydroformylierung homogen einphasig in organischen Lösungsmitteln durchgeführt, werden zweckmäßig wasserunlösliche, d.h. in organischen Lösungsmitteln lösliche organische Phosphorverbindungen verwendet.

Der Begriff homogenes Reaktionssystem steht für eine im wesentlichen aus Lösungsmittel, Katalysator, olefinisch ungesättigter Verbindung und Reaktionsprodukt zusammengesetzte homogene Lösung, wobei der Katalysator Rhodium und die phosphororganische Verbindung (komplexiert oder unkomplexiert) umfasst. Rhodium liegt im allgemeinen als Rhodium-Komplexverbindung vor, die die organischen Phosphorverbindungen als Liganden enthalten. Derartige Komplexverbindungen und ihre Herstellung sind bekannt (vgl. z. B. US 3 527 809, US 4148 830, US 4 247 486, US 4 283 562). Sie können als einheitliche Komplexverbindung oder auch als Gemisch unterschiedlicher Komplexverbindungen eingesetzt werden. Die Rhodium-Konzentration im Reaktionsmedium erstreckt sich über einen Bereich von etwa 1 bis etwa 1000 Gew.-ppm und beträgt vorzugsweise 10 bis 700 Gew.-ppm. Insbesondere wendet man Rhodium in Konzentrationen von 25 bis 500 Gew.-ppm, jeweils bezogen auf das homogene Reaktionsgemisch, an. Als Katalysator kann eine stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigem Phosphorliganden durchzuführen, der mit Rhodium keine Komplexverbindung eingeht. Der freie Phosphorligand kann der gleiche sein, wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Zu den bevorzugten phosphororganischen Verbindungen in den Rhodium-Komplexkatalysatoren beim Homogenverfahren zählen z.B. Triarylphosphine wie Triphenylphosphin, Trialkylphosphine wie Tri(n-octyl)phosphin, Trilaurylphosphin, Tri(cyclohexyl)phosphin, Alkylphenylphosphine, Cycloalkylphenylphosphine, organische Diphosphine und organische Diphosphite. Wegen seiner leichten Zugänglichkeit wird Triphenylphosphin besonders häufig angewandt. Üblicherweise beträgt das molare Verhältnis von Rhodium zu Phosphor 1:1 bis 1:300, jedoch kann der molare Anteil des Phosphors in Form organischer Phosphorverbindungen auch höher sein. Vorzugsweise setzt man Rhodium und organisch gebundenen Phosphor in molaren Verhältnissen von 1:3 bis 1:200 ein. Bei Anwendung von Triarylphosphinen haben sich insbesondere Rh/P-Molverhältnisse von 1:50 bis 1:150 bewährt. Werden Trialkylphosphine als Liganden eingesetzt, so beträgt das Molverhältnis von Rhodium zu Phosphor bevorzugt 1:3 bis 1:20.

Die einphasige Hydroformylierungsreaktion wird in der Regel in Gegenwart eines Lösungsmittels ausgeführt. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe wie Benzol, Toluol oder die Xylole. Andere gebräuchliche Lösungsmittel sind Paraffinöl, Ketone oder Ether. Als besonders geeignete Lösungsmittel haben sich die höhersiedenden Kondensationsverbindungen der Aldehyde erwiesen, die als Nebenprodukte bei der Hydroformylierung entstehen. Der Anteil des Lösungsmittels im Reaktionsmedium kann über einen weiten Konzentrationsbereich variiert werden und beträgt üblicherweise zwischen 20 und 90 Gew.-%, vorzugsweise 50 bis 80 Gew.-%, bezogen auf das Reaktionsgemisch.

Wird die Hydroformylierung hingegen heterogen zweiphasig in Gegenwart einer wässrigen Phase durchgeführt, so finden zweckmäßig wasserlösliche organische Phosphorverbindungen Anwendung.

Bevorzugt erfolgt die Herstellung der Aldehyde nach dem Heterogen-Zweiphasen-Flüssig-Flüssig-Verfahren in Gegenwart einer wäßrigen Phase, die mindestens eine Rhodiumverbindung sowie mindestens eine phosphororganische Verbindung als Katalysator gelöst enthält. Die Umsetzung der Olefine nach dem Heterogenverfahren ist z.B. in der DE 26 27 354 beschrieben. Das Verfahren ist charakterisiert durch das Vorliegen einer organischen Phase, die das olefinische Ausgangsmaterial und das Reaktionsprodukt enthält, und einer wäßrigen Phase, in der der Katalysator gelöst ist. Als Katalysatoren werden wasserlösliche Rhodium-Komplexverbindungen eingesetzt, die wasserlösliche organische Phosphor(III)-verbindungen als Liganden enthalten. Beispiele für wasserlösliche Phosphor(III)-verbindungen, die mit Rhodium Komplexverbindungen bilden, sind Triarylphosphine, Trialkylphosphine und arylierte bzw. alkylierte Diphosphine, deren organische Reste Sulfonsäuregruppen oder Carboxylgruppen enthalten. Ihre Herstellung und Anwendung ist z.B. aus DE 26 27 354, EP 0103810, EP 0163234 und EP 0571819 bekannt. Weitere Gruppen geeigneter Verbindungen sind sulfonierte oder carboxylierte organische Phosphite sowie heterocyclische Verbindungen des dreibindigen Phosphors (vgl. z.B. EP 0575785, EP 0640588).

Besonders bevorzugt finden wäßrige Hydroformylierungskatalysatorsysteme Anwendung, die Rhodium und Arylphosphine der allgemeinen Formel (I)

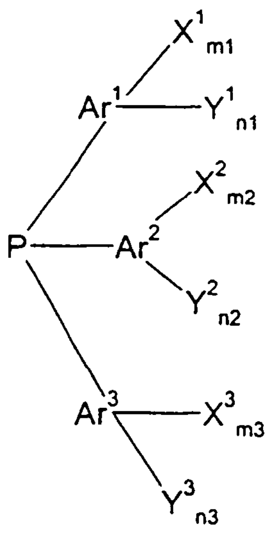

(I)

wobei $Ar^1$, $Ar^2$, $Ar^3$ jeweils eine Phenyl- oder Naphthyl-gruppe, $Y^1$,$Y^2$, $Y^3$ jeweils eine geradkettige oder verzweigte Alkylgruppe mit 1 bis 8 C-Atomen, eine geradkettige oder verzweigte Alkoxygruppe mit 1 bis 8 C-Atomen, ein Halogenatom, eine OH-, CN-, $NO_2$- oder $NR^1R^2$-Gruppe, in der $R^1$ und $R^2$ jeweils für eine geradkettige oder verzweigte Alkylgruppe mit 1 bis 8 C-Atomen stehen, bedeuten, $X^1$, $X^2$, $X^3$ für einen Carboxylat- ($-COO^-$-) und/oder einen Sulfonat-($-SO_3^-$-) Rest steht, m1, m2, m3 gleiche oder verschiedene Zahlen von 0 bis 3 sind, wobei mindestens eine Zahl m1, m2, m3 gleich oder größer als 1 ist, und n1, n2, n3 gleiche oder verschiedene ganze Zahlen von 0 bis 5 sind, komplexgebunden und gegebenenfalls im Überschuß enthalten.

Beispiele dieser wasserlöslichen organischen Phosphorverbindungen schließen ein: wasserlösliche Salze von mono-, di- oder trisulfonierten Triphenylphosphinverbindungen wie das Tris(m-sulfonatophenyl)phosphin-trinatriumsalz (TPPTS), das Bis(m-sulfonatophenyl)phenylphosphin-dikalium-dihydrat-Salz (TPPDS) sowie das Diphenylphosphinobenzol-3-sulfonsäure-natrium-Salz (TPPMS). Besonders bevorzugt ist das TPPTS.

Es wird angenommen, daß sich in Gegenwart von Synthesegas aus dem wasserlöslichen Arylphosphin der allgemeinen Formel (I) und der eingesetzten Rhodiumverbindung unter den Bedingungen der Hydroformylierungsreaktion katalytisch wirksame Rhodium-Komplexverbindungen bilden, die Kohlenmonoxid und das Arylphosphin der allgemeinen Formel als Liganden enthalten, und in der wäßrigen Phase gelöst vorliegen. Im allgemeinen enthält die wäßrige Katalysatorlösung einen Überschuß an den Arylphosphinen der allgemeinen Formel (I), die auch als Gemische vorliegen können.

Die organische Phase besteht im wesentlichen aus dem Einsatzolefin und/oder dem Reaktionsprodukt der Hydroformylierung, gegebenenfalls einem oder mehreren organischen Lösungsvermittlern sowie gegebenenfalls einem organischen Lösungsmittel. Bewährt haben sich kationische Lösungsvermittler der allgemeinen Formel $[A\text{-}N(R^3R^4R^5)]^+E^-$, in der A für einen geradkettigen oder verzweigten Alkylrest mit 6 bis 25 Kohlenstoffatomen steht, $R^3$, $R^4$ und $R^5$ gleich oder verschieden sind und geradkettige oder verzweigte Alkylreste mit 1 bis 4 Kohlenstoffatomen bedeuten und E insbesondere für Sulfat, Tetrafluoroborat, Acetat, Methosulfat, Benzolsulfonat, Alkylbenzolsulfonat, Toluolsulfonat, Lactat oder Citrat steht.

Arbeitet man in Gegenwart eines Lösungsmittels, so verwendet man inerte aliphatische Verbindungen, wie Alkane, zum Beispiel $C_5$-$C_9$-Alkane wie Cyclohexan und n-Pentan, oder aromatische Verbindungen, wie Toluol, Xylol Ethylbenzol, Mesitylen oder Chlorbenzol.

Das Volumenverhältnis von organischer Phase zu der wäßrigen Katalysatorlösung liegt im allgemeinen zwischen 5 : 1 und 1 : 5. Bevorzugt ist ein Bereich von 3 : 1 bis 1: 2. Ein höherer Anteil der organischen Phase führt im allgemeinen zu einer Verlangsamung der Reaktionsgeschwindigkeit während man bei einem geringeren Anteil der organischen Phase einen höheren Rhodiumaustrag in die organische Phase beobachtet.

Zu Beginn der Hydroformylierungsreaktion gelangt das Rhodium entweder als Metall oder als Verbindung zum Einsatz. In metallischer Form verwendet man es entweder als feinverteilte Partikel oder in dünner Schicht auf einem Träger, wie Aktivkohle, Calciumcarbonat, Aluminiumsilikat, Tonerde niedergeschlagen. Als Rhodiumverbindungen eignen sich Salze aliphatischer Mono- und Polycarbonsäuren, wie Rhodium-2-ethylhexanoat, Rhodiumacetat, Rhodiumoxalat, Rhodiumpropionat oder Rhodiummalonat. Weiterhin können Rhodiumsalze anorganischer Wasserstoff- und Sauerstoffsäuren, wie Rhodiumnitrat oder Rhodiumsulfat, die verschiedenen Rhodiumoxide oder auch Rhodiumcarbonylverbindungen wie $Rh_3(CO)_{12}$ oder $Rh_6(CO)_{16}$ oder Komplexverbindungen des Rhodiums, z.B. Cyclooctadienyl-rhodiumverbindungen oder Rhodiumacetylacetonat, eingesetzt werden. Rhodiumhalogenverbindungen kommen wegen des korrosiven Verhaltens der Halogenidionen weniger in Betracht.

Bevorzugt werden Rhodiumoxid und insbesondere Rhodiumacetat und Rhodium-2-ethylhexanoat.

Die Rhodiumkonzentration beträgt zweckmäßig 150 bis 800, vorzugsweise 200 bis 400 mg Rhodium, bezogen auf 1 Kilogramm wäßriger Katalysatorphase.

Der pH-Wert der wäßrigen Katalysatorlösung beim Zweiphasenverfahren liegt im allgemeinen oberhalb von 3, vorzugsweise im Bereich von 5 bis 8. Gegebenfalls ist der Einsatz einer wäßrigen Pufferlösung zur Einhaltung eines bestimmten pH-Wertes erforderlich. Als Pufferlösungen werden wäßrige Lösungen von Alkali- oder Erdalkalisalzen, wie zum Beispiel von Phosphaten, Hydrogenphosphaten, Carbonaten, Hydrogencarbonaten, Boraten, Acetaten eingesetzt.

Es hat sich auch bei der heterogenen Hydroformylierung bewährt, Rhodium und die Arylphosphine nicht in stöchiometrischem Verhältnis, also entsprechend der chemischen Zusammensetzung der Rhodium-Komplexverbindung zu verwenden, die sich im Laufe des Präformierungs- oder Hydroformylierungsschrittes bildet, sondern die Arylphosphine im Überschuß einzusetzen. Dabei kann das Molverhältnis von Rhodium zu dem Arylphosphin insbesondere dem der allgemeinen Formel (I), auch ausgedrückt als das Verhältnis von Rhodium zu Phosphor im Arylphosphin, in weiten Grenzen variieren. Im allgemeinen verwendet man je mol Rhodium etwa 1 bis 5000 mol Phosphor. Bevorzugt wird ein Molverhältnis Phosphor (III) zu Rhodium von 1 bis 200 und insbesondere von 50 bis 150. Den eigentlichen Hydroformylierungskatalysator plus überschüssiges Arylphosphin bezeichnet man auch als Katalysatorsystem.

Die Umsetzung der Olefine mit Wasserstoff und Kohlenmonoxid (Synthesegas) in der Hydroformylierung erfolgt sowohl homogen als auch heterogen im allgemeinen bei einem Druck von 0,1 bis 50 MPa, bevorzugt von 1 bis 50 MPa, bevorzugter 1 bis 20 MPa, noch bevorzugter von 2 bis 20 und ganz besonders bevorzugt bei 3 bis 8 MPa und bei einer Temperatur von 20 bis 200°C, bevorzugt 40 bis 200°C, bevorzugter 40 bis 150°C, noch bevorzugter bei 80 bis 150°C und besonders bevorzugt von 110 bis 130°C. Hohe Drücke fördern die Aktivität des Katalysatorsystems vermindern jedoch das Selektivitätsverhältnis von geradkettigem Aldehyd zu verzweigtem Aldehyd. Bei zu niedrigen Temperaturen ist die Reaktionsgeschwindigkeit unannehmbar langsam, wohingegen bei zu hohen Temperaturen, die erfindungsgemäß ja gerade vermieden werden sollen, die Katalysatorschädigung ein nicht akzeptables Ausmaß erreicht. Die optimalen Hydroformylierungsbedingungen, insbesondere die Rhodiumkonzentration, der Druck und die Temperatur hängen von der einzusetzenden olefinischen Verbindung ab. Reaktive Olefine, wie zum Beispiel Propylen, Buten-1, Penten-1, Hexen-1, Butadien-1,3, erfordern nur geringe Rhodiumkonzentrationen, niedrige Drücke und niedrige Temperaturen. Hingegen benötigt die Umsetzung reaktionsträger, olefinischer Verbindungen, zum Beispiel von Buten-2 und anderer Olefine mit innenständiger Doppelbindung, höhere Rhodiumkonzentrationen, Drücke und Temperaturen.

Auch zu Beginn der Hydroformylierung kann der Katalysator dem Reaktionsgemisch präformiert zugesetzt werden (s. z.B. EP-A-0246475). Dazu wird zunächst die wässrige, Rhodium, vorzugsweise als Rhodium-2-ethylhexanoat und die Arylphosphine, vorzugsweise der allgemeinen Formel (I), enthaltende Lösung mit Synthesegas bei erhöhter Temperatur und erhöhtem Druck über einen Zeitraum von 0,1-10 Stunden behandelt. Im allgemeinen entsprechen die Bedingungen der Präformierung denen der eigentlichen Hydroformylierung in Gegenwart der umzusetzenden olefinischen Verbindung, die sich an den Präformierungsschritt anschließt.

Die Zusammensetzung des Synthesegases, d.h. das Verhältnis von Kohlenmonoxid zu Wasserstoff kann in weiten Grenzen variiert werden. Im allgemeinen setzt man Synthesegas ein, in dem das Volumenverhältnis von Kohlenmonoxid zu Wasserstoff etwa 1 : 1 beträgt oder von diesem Wert nur wenig abweicht. Im allgemeinen verwendet man während der Präformierung für den Ausgangskatalysator und während der Hydroformylierung Synthesegas gleicher Zusammensetzung.

Die Hydroformylierung kann sowohl absatzweise als auch kontinuierlich durchgeführt werden. Bei der diskontinuierlichen Fahrweise wird nach Abschluß der Reaktion das Hydroformylierungsgemisch durch Druckentspannung von Kohlenmonoxid und Wasserstoff befreit.

Nach beendeter Hydroformylierungsreaktion liegt bei der Durchführung der Hydroformylierung in Gegenwart einer wässrigen Phase eine flüssige organische, aldehydhaltige obere Phase und eine wäßrige den Katalysator enthaltende untere Phase vor, die durch einfache Phasentrennung voneinander getrennt werden können. Nach erfolgter Phasentrennung wird die wäßrige katalysatorhaltige Phase, gegebenfalls nach Ergänzung mit frischem, erfindungsgemäß vorbehandelten Katalysator, wie unten beschrieben, wieder in den Hydroformylierungsprozeß zurückgeschleust.

Das erfindungsgemäße Hydroformylierungsverfahren zeichnet sich dadurch aus, dass während des Verfahrens zusätzliches Rhodium in der Form eines Umsetzungsproduktes zugesetzt wird, dessen Herstellung die Umsetzung von einem oder mehreren Rhodiumsalzen einer organischen Verbindung, ausgewählt aus der Gruppe von Rhodiumsalzen, die aus Rhodium(III)-salzen von Beta-Diketonen und Rhodiumsalzen von Carbonsäuren besteht, in Gegenwart von organischen Phosphorverbindungen oder deren Salzen mit Kohlenmonoxid und Wasserstoff umfaßt.

Die ergänzende Zugabe des Rhodiumumsetzungsproduktes kann zu irgend einem Zeitpunkt nach dem Beginn der Hydroformylierungsreaktion erfolgen. Zweckmäßig erfolgt sie dann, wenn ein Aktivitätsverlust, der sich durch einen

reduzierten Aldehydaustrag bemerkbar macht, eingetreten ist, oder aber auch rechtzeitig bevor ein solcher Aktivitätsverlust einsetzt, wenn dieser aufgrund der bekannten Standzeiten des ursprünglich eingesetzten Katalysators zu erwarten ist.

Der Zeitpunkt, in dem die Zugabe weiteren Rhodiums in der Form des erfindungsgemäß verwendeten Umsetzungsproduktes stattfindet, und die Mengen des Umsetzungsproduktes, die dabei jeweils zugesetzt werden, unterliegen grundsätzlich keiner Einschränkung. Sie hängen von der Art und Weise ab, wie der Reaktor betrieben wird. So ist es z.B. möglich, mit der Rhodiumergänzung zu einem frühen Zeitpunkt in kleinen Mengen zu beginnen und den Umsatz so auf dem Ausgangsniveau zu halten, oder man setzt nach einem längeren Zeitraum eine größere Menge Rhodium zu. Werden z.B. nach einem längeren Zeitraum, größere Mengen des Umsetzungsproduktes zugesetzt, so erfolgt die Zugabe z.B. etwa 20 bis 30 Tage nach Beginn der Hydroformylierungsreaktion. Werden in kürzeren Abständen kleinere Mengen des Umsetzungsproduktes zugesetzt, so kann mit der Zugabe bereits etwa nach 4 bis 5 Tagen begonnen und diese Zugabe in diesen Abständen wiederholt werden.

Die Zugabe des Rhodiumumsetzungsproduktes erfolgt in der Regel so, daß eine Lösung des Umsetzungsproduktes ohne Aufarbeitung, wie sie bei der Vorbehandlung erhalten wird, direkt in den Hydroformylierungsreaktor gegeben wird. Wird die Hydroformylierung homogen geführt, so wird die erhaltene organische Lösung des Umsetzungsproduktes zugesetzt. Wird die Hydroformylierung heterogen geführt, so wird eine wässrige Lösung des Umsetzungsproduktes zugesetzt. Prinzipiell ist es bei heterogener Reaktionsführung auch möglich, das Rhodiumumsetzungsprodukt zu einer aus dem Reaktor ausgeschleusten, den Katalysator enthaltenden Phase zu geben, die anschließend wieder in den Reaktor zurückgeführt wird. Dies ist jedoch nicht bevorzugt.

Die Menge des Rhodiumumsetzungsproduktes, die bei jeder Zugabe hinzugefügt wird, hängt wie bereits dargelegt, von der Art und Weise ab, wie der Reaktor betrieben wird. Werden kleine Mengen in kürzeren Zeitabständen zugesetzt, so beträgt die Menge des Rhodiumumsetzungsproduktes, bezogen auf das Rhodium im Rhodiumumsetzungsprodukt, mindestens etwa 1 Gew.-%, bezogen auf das bereits im Reaktor vorhandene Rhodium. Werden in längeren Zeitabständen größere Mengen des Rhodiumumsetzungsproduktes nachgegeben, so werden je ergänzender Zugabe, bezogen auf das Rhodium im Rhodiumumsetzungsprodukt maximal etwa 30% bezogen auf das bereits im Reaktor vorhandenen Rhodium nachgesetzt.

Für die Herstellung des Rhodiumumsetzungsproduktes, das in die laufende Hydroformylierung zur Ergänzung hinzugegeben wird, verwendet man ein oder mehrere Rhodiumsalze einer organischen Verbindung, ausgewählt aus der Gruppe von Rhodiumsalzen, die aus Rhodium(III)-salzen von Beta-Diketonen und Rhodiumsalzen von Carbonsäuren besteht.

Die Carbonsäuren können ein- oder mehrbasisch, geradkettig oder verzweigt sein. Sowohl Salze gesättigter oder ungesättigter aliphatischer Säuren als auch Salze aromatischer Säuren sind geeignet. Die Herstellung der Salze erfolgt z.B. durch Umsetzung wäßriger Rhodiumsalzlösungen wie Rhodium(III)-nitrat oder Rhodium(III)-sulfat mit den wäßrigen Lösungen von Salzen der organischen Säuren oder durch Umsetzung von Rhodiumoxid bzw. Rhodiumoxid-Hydraten mit den freien Säuren.

Besonders bevorzugt werden bei der Herstellung des genannten Umsetzungsproduktes ein oder mehrere Rhodiumsalze, ausgewählt aus der Gruppe von Rhodiumsalzen, die besteht aus Rhodium(III)-acetylacetonat und Rhodium-(bevorzugt Rhodium(III))salzen aliphatischer Monocarbonsäuren mit 2 bis 18, bevorzugt 2 bis 12 noch bevorzugter 2 bis 10 Kohlenstoffatomen, mit Kohlenmonoxid und Wasserstoff umgesetzt.

Besonders geeignet sind die Rhodiumsalze gesättigter Monocarbonsäuren mit 2 bis 10 Kohlenstoffatomen. Beispiele für diese Säuren sind Essigsäure, Propionsäure, n-Buttersäure, i-Buttersäure, Pentansäure, Hexansäure, 2-Ethylhexansäure.

Wird die Hydroformylierung als Zweiphasenreaktion im Gegenwart einer wäßrigen Phase durchgeführt, so ist Rhodiumacetat (Rhodium-(III)-acetat oder Rhodium(II)-acetat, bevorzugt Rhodium-(III)-acetat) das am meisten bevorzugte Ausgangsmaterial. Wird die Hydroformylierung hingegen einphasig in einem organischen Lösungsmittel durchgeführt, so ist Rhodium(II)-2-ethylhexanoat das am meisten bevorzugte Ausgangsmaterial.

Die Herstellung des genannten Umsetzungsproduktes kann im Prinzip unter den Druck- und Temperaturbedingungen der Hydroformylierung erfolgen.

Die Zusammensetzung des $CO/H_2$-Gemisches kann in weiten Bereichen variiert werden. Es ist möglich, sowohl kohlenmonoxidreiche als auch wasserstoffreiche Gemische zu verwenden. Üblicherweise setzt man Mischungen ein, die Kohlenmonoxid und Wasserstoff etwa im Volumenverhältnis 1 : 1 enthalten, d.h. eine Zusammensetzung aufweisen, wie sie auch in der anschließenden Hydroformylierung angewendet wird. Besonders bevorzugt erfolgt die Umsetzung der Rhodiumverbindung in Gegenwart von Kohlenmonoxid und Wasserstoff bei einem Druck von 0,1 bis 10 MPa und bei einer Temperatur von 50 bis 200°C. Noch bevorzugter sind 100 bis 150°C und 1 bis 5 MPa, womit ein optimaler Verlauf der Reaktion sichergestellt ist.

Die Herstellung des Rhodiumumsetzungsproduktes erfolgt in Gegenwart einer phosphororganischen Verbindung, bevorzugt in Gegenwart der gleichen phosphororganischen Verbindung wie sie in der laufenden Hydroformylierung verwendet wird. D.h., daß bei Einsatz des Rhodiumumsetzungsproduktes für die homogene einphasige Hydroformylierung das Rhodiumsalz im allgemeinen in Gegenwart von wasserunlöslichen Phosphinen, beispielsweise Alkyl- oder Arylphosphinen, wie z.B. Triphenylphosphin umgesetzt wird. Bei der Verwendung des Rhodiumumsetzungsproduktes in der Zweiphasen-Flüssig-Flüssig-Hydroformylierung in Gegenwart einer wäßrigen Phase erfolgt die Herstellung des Rhodiumumsetzungsproduktes in Gegenwart der an sich bekannten wasserlöslichen Phosphine, wie insbesondere TPPTS.

Das Verhältnis zwischen Rhodium und dem Phosphin bei der Herstellung des nachzusetzenden Rhodiumumsetzungsproduktes beträgt in beiden Fällen ausgedrückt als Verhältnis von Phosphor(III) zu Rhodium bevorzugt 5 : 1 - 100 : 1, besonders bevorzugt 10 : 1 - 30 : 1.

Da für die Herstellung des nachzusetzenden Rhodiumumsetzungsproduktes im allgemeinen die gleichen phosphororganischen Verbindungen, insbesondere die gleichen Phosphine wie für die laufende Hydroformylierung verwendet werden, gilt für die bevorzugten phosphororganischen Verbindungen das oben zu den Hydroformylierungsverfahren gesagte. Insbesondere sind also wasserunlösliche Alkyl- oder Arylphosphine, wie z.B. Triphenylphosphin bei der homogenen einphasigen Hydroformylierung bevorzugt, während wasserlösliche Sulfonsäuregruppen-enthaltende Phosphine wie z.B. insbesondere das TPPTS (Triphenylphosphintrisulfonsäuretrinatriumsalz) für die zweiphasige Hydroformylierung in Gegenwart einer wässrigen Phase bevorzugt sind.

Erfolgt der Einsatz des erfindungsgemäß verwendeten Umsetzungsproduktes bei der Zweiphasenhydroformylierung, so wird das Umsetzungsprodukt zweckmäßig in einer wäßrigen Phase, die wasserlösliches Phosphin enthält, bereitgestellt. Die Herstellung einer wäßrigen Lösung des Umsetzungsproduktes kann im Prinzip auf zwei verschiedene Arten erfolgen, je nachdem, ob als Ausgangsmaterial eine wässerlösliche Rhodiumverbindung oder eine wasserunlösliche Rhodiumverbindung verwendet wird. In einer bevorzugten Ausführungsform wird als wasserlösliche Rhodiumausgangsverbindung Rhodiumacetat, besonders bevorzugt Rhodium(III)-acetat in einer wäßrigen Phase in Gegenwart eines wasserlöslichen Phosphins, insbesondere TPPTS mit dem $CO/H_2$-Gemisch umgesetzt. Es ist aber auch möglich, eine organische Lösung eines wasserunlöslichen Rhodiumsalzes, wie z.B. Rhodium(II)-2-ethylhexanoat in Gegenwart einer wäßrigen Phase, die das wasserlösliche Phosphin, insbesondere TPPTS, enthält, mit dem $CO/H_2$-Gemisch umzusetzen. Das Rhodium wird bei dieser Verfahrensweise als wasserlöslicher Komplex in die wässrige Phase überführt, die dann in den Hydroformylierungsreaktor gegeben wird.

Als organische Lösungsmittel werden zum Beispiel aliphatische, cycloaliphatische oder aromatische Kohlenwasserstoffe eingesetzt. Besondere Anforderungen an die physikalischen Eigenschaften der Kohlenwasserstoffe werden nicht gestellt. Sie müssen aber selbstverständlich frei von solchen Verunreinigungen sein, die das katalytisch wirksame Rhodium desaktivieren. Die Konzentration des Rhodiums in dem Kohlenwasserstoff ist nicht kritisch. Zweckmäßig setzt man mäßig konzentrierte Lösungen ein, insbesondere solche, die mindestens 3000 mg Rhodium je 1 Liter Lösung enthalten. Es ist nicht erforderlich, einheitliche Kohlenwasserstoffe einzusetzen. Auch Gemische verschiedener Kohlenwasserstoffe sind als Lösungsmittel für die Rhodiumsalze geeignet. Bewährt haben sich Pentan, Hexan, Benzinfraktionen des Rohöls, Toluol und Xylole.

Bei der Umsetzung einer wäßrigen Lösung eines wasserlöslichen Rhodiumsalzes ist die Konzentration des Rhodiums in der wäßrigen Phase ebenfalls nicht kritisch. Zweckmäßig versucht man relativ hohe Konzentrationen des Rhodiums zu verwenden, so daß mindestens etwa 3000 mg Rhodium je Liter wäßriger Lösung vorhanden sein sollten.

Die Herstellung des Rhodiumumsetzungsproduktes dauert je nach Reaktionsbedingungen etwa 0,5 bis 10, meist etwa 4 bis 8 Stunden.

Nach Abschluß der Umsetzung bzw. Vorbehandlung wird das entstandene Umsetzungsprodukt im allgemeinen ohne weitere Aufarbeitung direkt in den Hydroformylierungsreaktor gegeben.

Die Erfindung betrifft ferner die Verwendung eines Umsetzungsproduktes auf der Basis von Rhodium, dessen Herstellung die Umsetzung von einem oder mehreren Rhodiumdsalzen einer organischen Verbindung, ausgewählt aus der Gruppe von Rhodiumsalzen, die aus Rhodium(III)-salzen von Beta-Diketonen und Rhodiumsalzen von Carbonsäuren besteht, in Gegenwart von organischen Phosphorverbindungen oder deren Salzen mit Kohlenmonoxid und Wasserstoff umfasst, zur Erhöhung der Aktivität eines Katalysators auf der Basis von Rhodium in einem Hydroformylierungsverfahren, worin das genannte Umsetzungsprodukt dem Hydroformylierungsverfahren hinzugesetzt wird. Bezüglich der Einzelheiten dieser Verwendung kann auf die vorstehenden Ausführungen verwiesen werden.

In den folgenden Beispielen wird die Erfindung näher erläutert, sie ist aber selbstverständlich nicht auf die beschriebenen Ausführungsformen beschränkt.

Experimenteller Teil

Zur Beschreibung der Wirksamkeit der jeweiligen Katalysatorlösung wurden die nachstehend definierten Größen "Aktivität" und "Produktivität" ermittelt.

Aktivität: mol (n+i) Aldehyd/g-Atom Rhodium x Minute

Produktivität: kg (n+i) Aldehyd / Liter Katalysatorlösung x Stunde

Für die nachfolgend beschriebenen Versuche wurde eine wäßrige, TPPTS enthaltende Katalysatorlösung aus dem laufenden Betrieb (Zweiphasenverfahren) eingesetzt und diese für alle drei Versuche als Grundlage verwendet

Beispiel 1: Vergleichsversuch mit einer wäßrigen, TPPTS enthaltenden Katalysatorlösung aus dem laufenden Betrieb

Zur Ermittlung des Nullniveaus wurde die Katalysatorlösung ohne weitere Zusätze in einen semikontinuierlich arbeitenden Versuchsautoklav eingesetzt, welcher wie folgt betrieben wurde. Einem mit Rührer ausgerüsteten 0,21 Edelstahlautoklav wurden Propylen und ein aus gleichen Volumenteilen bestehendes $CO/H_2$-Gemisch in einer solchen Menge zugeleitet, dass 10 Nl/h (1 Normliter (Nl) ist gleich 1 Liter bei einem technischen Druck von 1 atm und einer Temperatur von 20°C) Abgas aus dem Reaktor entnommen wurde. Gleichzeitig wurden je Stunde 300 ml wäßrige Katalysatorlösung im Kreis durch den Reaktor geführt. Die Hydroformylierungen wurden über mehrere Tage durchgeführt, wobei der Autoklav 8 Stunden am Tag betrieben wurde. Die übrigen Reaktionsparameter sind der Tabelle 1 zu entnehmen. Die Ergebnisse des Nulltestes sind ebenfalls der folgenden Tabelle zu entnehmen.

Tabelle 1: Propylen-Hydroformylierung nach dem Zweiphasenverfahren ohne nachfolgende Rhodiumergänzung.

| **Versuchsdauer [h]** | 8 | 16 | 32 | 64 |
|---|---|---|---|---|
| **Temperatur [°C]** | 130 | 130 | 130 | 130 |
| **Druck [MPa]** | 5 | 5 | 5 | 5 |
| **Rh-Gehalt [mg/kg]** | 271 | 271 | 270 | 269 |
| **P(III) Gehalt [mmol/kg]** | 170 | 170 | 169 | 167 |
| **Ligand/Rh** | 65 | 65 | 65 | 65 |
| **C3-Einsatz [g/h]** | 40 | 40 | 40 | 40 |
| **Aktivität [molC4-al/molRh*min]** | 14,33 | 14,13 | 14,45 | 14,56 |
| **Produktivität [kg C4-al/l Kat.lsg.*h]** | 0,213 | 0.210 | 0,215 | 0,216 |
| **n/i-Verhältnis** | 92/8 | 92/8 | 92/8 | 92/8 |

Beispiel 2: Zusatz von 30 ppm Rhodium-Acetat zu der im Beispiel 1 getesteten Katalysatorlösung aus dem laufenden Betrieb.

Die Ergänzung von 30 ppm Rhodium-Acetat zu der im Beispiel 1 eingesetz- , ten Katalysatorlösung erfolgte unter analogen Katalysebedingungen. Nach Ermittlung des Nullniveaus wurde einmalig ergänzt. Die Ergänzung des Rhodiums erfolgte während der Hydroformylierung über den Katalysatorkreislauf. Die Hydroformylierungen wurden über mehrere Tage durchgeführt, wobei der Autoklav 8 Stunden am Tag betrieben wurde. Die übrigen Reaktionsparameter sind der Tabelle 2 zu entnehmen. Das Ergebnis des Nulltestes ist ebenfalls der folgenden Tabelle zu entnehmen. Dieses Ergebnis ist mit den Resultaten aus dem Beispiel 1 vergleichbar.

Tabelle 2: Propylen-Hydroformylierung nach dem Zweiphasenverfahren mit nachträglichen, nichtpräformierter Rhodiumzugabe

| **Versuchsdauer [h]** | Nulltest | 8<br>Nach Zusatz von 30 ppm Rh-Acetat | 16 | 32 | 64 |
|---|---|---|---|---|---|
| **Temperatur [°C]** | 130 | 130 | 130 | 130 | 130 |
| **Druck [MPa]** | 5 | 5 | 5 | 5 | 5 |
| **Rh-Gehalt [mg/kg]** | 271 | 300 | 299 | 301 | 300 |
| **P(III) Gehalt [mmol/kg]** | 170 | 170 | 171 | 171 | 169 |
| **Ligand/Rh** | 65 | 58 | 59 | 59 | 58 |
| **C3-Einsatz [g/h]** | 40 | 40 | 40 | 40 | 40 |
| **Aktivität [molC4-al/ molRh*min]** | 14,20 | 15,13 | 15,01 | 15,29 | 15,18 |
| **Produktivität [kg C4-al/l Kat.lsg.*h]** | 0,211 | 0,225 | 0,221 | 0,229 | 0,226 |
| **n/i-Verhältnis** | 92/8 | 92/8 | 92/8 | 92/8 | 92/8 |

Beispiel 3: Zusatz von 30 ppm präformiertem Rhodium zu der im Beispiel 1 getesteten Katalysatorlösung aus dem laufenden Betrieb.

Die Ergänzung von 30 ppm präformiertem Rhodium zu der im Beispiel 1 eingesetzten Katalysatorlösung erfolgte unter analogen Katalysebedingungen. Nach Ermittlung des Nullniveaus wurde einmalig ergänzt. Hierzu wurden zuvor in einem 150 ml Autoklav eine Lösung aus 61 mmol TPPTS und 6,1 mmol Rhodium-Acetat in 138 g Wasser eingesetzt, was einer wäßrigen Katalysatormenge von 140 g entsprach. Diese Lösung wurde 3 Stunden bei 120°C und 5 MPa Synthesegasdruck (Zusammensetzung $H_2$:CO = 1:1) präformiert. Zur Ergänzung wurden anschließend 2,1 g der präformierten Lösung während der Hydroformylierung über den Katalysatorkreislauf zugesetzt. Die Hydroformylierungen wurden über mehrere Tage durchgeführt, wobei der Autoklav 8 Stunden am Tag betrieben wurde. Die übrigen Reaktionsparameter sind der Tabelle 3 zu entnehmen. Das Ergebnis des Nulltestes ist ebenfalls der folgenden Tabelle zu entnehmen. Dieses ist mit den Resultaten aus dem Beispiel 1 vergleichbar.

Tabelle 3: Propylen-Hydroformylierung nach dem Zweiphasenverfahren mit nachträglicher präformierter Rhodiumzugabe.

| **Versuchsdauer [h]** | Nulltest | 8<br>Nach Zusatz von 30 ppm präformiertem Rhodium | 16 | 32 | 64 |
|---|---|---|---|---|---|
| **Temperatur [°C]** | 130 | 130 | 130 | 130 | 130 |
| **Druck [MPa]** | 5 | 5 | 5 | 5 | 5 |
| **Rh-Gehalt [mg/kg]** | 271 | 300 | 299 | 301 | 300 |
| **P(III) Gehalt [mmollkg]** | 170 | 173 | 174 | 173 | 174 |
| **Ligand/Rh** | 65 | 59 | 60 | 59 | 59 |

(fortgesetzt)

| Versuchsdauer [h] | Nulltest | 8<br>Nach Zusatz von 30 ppm präformiertem Rhodium | 16 | 32 | 64 |
|---|---|---|---|---|---|
| C3-Einsatz [g/h] | 40 | 40 | 40 | 40 | 40 |
| Aktivität [molC4-al/ molRh*min] | 14,17 | 16,58 | 16,75 | 16,70 | 16,64 |
| Produktivität [kg C4-al/l Kat.lsg. *h] | 0,210 | 0,246 | 0,251 | 0,250 | 0,249 |
| n/i-Verhältnis | 92/8 | 92/8 | 92/8 | 92/8 | 92/8 |

Wie aus den Beispielen 1-3 ersichtlich, lassen sich durch nachträgliche Rhodiumzugabe in präformierter Form Aktivität und Produktivität des Katalysatorsystems gegenüber einer Rhodiumzugabe in nichtpräformierter Form deutlich steigern.

**Patentansprüche**

1. Verfahren zur Herstellung von Aldehyden durch Umsetzung olefinisch ungesättigter Verbindungen mit Kohlenmonoxid und Wasserstoff bei Temperaturen von 20 bis 200°C und Drücken von 0,1 bis 50 MPa in flüssiger Phase in Gegenwart eines Katalysators auf der Basis von Rhodium, worin während des Verfahrens zusätzliches Rhodium in der Form eines Umsetzungsproduktes zugesetzt wird, dessen Herstellung die Umsetzung von einem oder mehreren Rhodiumsalzen einer organischen Verbindung, ausgewählt aus der Gruppe von Rhodiumsalzen, die aus Rhodium(III)-salzen von Beta-Diketonen und Rhodiumsalzen von Carbonsäuren besteht, in Gegenwart von organischen Phosphorverbindungen oder deren Salzen mit Kohlenmonoxid und Wasserstoff umfaßt.

2. Verfahren nach Anspruch 1, worin die Umsetzung der olefinisch ungesättigten Verbindungen in Gegenwart einer flüssigen wässrigen und einer flüssigen organischen Phase durchgeführt wird.

3. Verfahren nach Anspruch 1 oder 2, worin die Umsetzung der olefinisch ungesättigten Verbindungen in Gegenwart von organischen Phosphorverbindungen oder deren Salzen durchgeführt wird.

4. Verfahren nach irgend einem der Ansprüche 1 bis 3, worin die Herstellung des genannten Umsetzungsproduktes bei einem Druck von 0,1 bis 10 MPa und bei einer Temperatur von 50 bis 200°C durchgeführt wird.

5. Verfahren nach irgend einem der Ansprüche 1 bis 4, worin bei der Herstellung des genannten Umsetzungsproduktes ein oder mehrere Rhodiumsalze, ausgewählt aus der Gruppe von Rhodiumsalzen, die besteht aus Rhodium(III)-acetylacetonat und Rhodiumsalzen aliphatischer Monocarbonsäuren mit 2 bis 18 Kohlenstoffatomen, mit Kohlenmonoxid und Wasserstoff umgesetzt werden.

6. Verfahren nach irgend einem der Ansprüche 1 bis 5, worin bei der Herstellung des genannten Umsetzungsproduktes Rhodium-2-ethylhexanoat oder Rhodiumacetat mit Kohlenmonoxid und Wasserstoff umgesetzt wird.

7. Verwendung eines Umsetzungsproduktes auf der Basis von Rhodium, dessen Herstellung die Umsetzung von einem oder mehreren Rhodiumsalzen einer organischen Verbindung, ausgewählt aus der Gruppe von Rhodiumsalzen, die aus Rhodium(III)-salzen von Beta-Diketonen und Rhodiumsalzen von Carbonsäuren besteht, in Gegenwart von organischen Phosphorverbindungen oder deren Salzen mit Kohlenmonoxid und Wasserstoff umfasst, zur Erhöhung der Aktivität eines Katalysators auf der Basis von Rhodium in einem Hydroformylierungsverfahren, worin das genannte Umsetzungsprodukt dem Hydroformylierungsverfahren hinzugesetzt wird.

## Claims

1. A process for preparing aldehydes by reaction of olefinically unsaturated compounds with carbon monoxide and hydrogen at temperatures of from 20 to 200°C and pressures of from 0.1 to 50 MPa in the liquid phase in the presence of a catalyst based on rhodium, which comprises, during the process, adding supplementary rhodium in the form of a reaction product prepared by reacting one or more rhodium salts of an organic compound selected from the group of rhodium salts consisting of rhodium(III) salts of beta-diketones and rhodium salts of carboxylic acids, in the presence of organophosphorus compounds or their salts with carbon monoxide and hydrogen.

2. The process as claimed in claim 1, wherein the reaction of the olefinically unsaturated compounds is carried out in the presence of a liquid aqueous and a liquid organic phase.

3. The process as claimed in claim 1 or 2, wherein the reaction of the olefinically unsaturated compounds is carried out in the presence of organophosphorus compounds or their salts.

4. The process as claimed in any of claims 1 to 3, wherein the preparation of said reaction product is carried out at a pressure of from 0.1 to 10 MPa and at a temperature of from 50 to 200°C.

5. The process as claimed in any of claims 1 to 4, wherein said reaction product is prepared by reacting one or more rhodium salts selected from the group of rhodium salts consisting of rhodium(III) acetylacetonate and rhodium salts of aliphatic monocarboxylic acids having from 2 to 18 carbon atoms, with carbon monoxide and hydrogen.

6. The process as claimed in any of claims 1 to 5, wherein said reaction product is prepared by reacting rhodium 2-ethyl hexanoate or rhodium acetate with carbon monoxide and hydrogen.

7. The use of a reaction product based on rhodium which is prepared by reacting one or more rhodium salts of an organic compound selected from the group of rhodium salts consisting of rhodium(III) salts of beta-diketones and rhodium salts of carboxylic acids, in the presence of organophosphorus compounds or their salts with carbon monoxide and hydrogen for increasing the activity of a catalyst based on rhodium in a hydroformylation process, which comprises adding said reaction product to said hydroformylation process.

## Revendications

1. Procédé de préparation d'aldéhydes par réaction de composés à insaturation oléfinique avec du monoxyde de carbone et de l'hydrogène à des températures de 20 à 200°C et sous des pressions de 0,1 à 50 MPa, en phase liquide, en présence d'un catalyseur à base de rhodium, où, pendant le procédé, on ajoute une quantité supplémentaire de rhodium sous forme d'un produit de réaction, dont la préparation comprend la réaction d'un ou plusieurs sels de rhodium d'un composé organique choisi dans le groupe des sels de rhodium constitué des sels de rhodium (III) de bêta-dicétones et de sels de rhodium d'acides carboxyliques, en présence de composés organosphosphorés ou de leurs sels, avec du monoxyde de carbone et de l'hydrogène.

2. Procédé selon la revendication 1, dans lequel la réaction des composés à insaturation oléfinique est effectuée en présence d'une phase aqueuse liquide et d'une phase organique liquide.

3. Procédé selon la revendication 1 ou 2, dans lequel la réaction des composés à insaturation oléfinique est effectuée en présence de composés organophosphorés ou de leurs sels.

4. Procédé selon l'une quelconque des revendications 1 à 3, dans lequel la préparation du produit de réaction mentionné est effectuée sous une pression de 0,1 à 10 MPa et à une température de 50 à 200°C.

5. Procédé selon l'une quelconque des revendications 1 à 4, dans lequel, lors de la préparation du produit de réaction mentionné, on fait réagir avec du monoxyde de carbone et de l'hydrogène un ou plusieurs sels de rhodium, choisi dans le groupe des sels de rhodium qui est constitué de l'acétylacétonate de rhodium(III) et des sels de rhodium d'acides monocarboxyliques aliphatiques ayant 2 à 18 atomes de carbone.

6. Procédé selon l'une quelconque des revendications 1 à 5, dans lequel, lors de la préparation du produit de réaction mentionné, on fait réagir avec du monoxyde de carbone et de l'hydrogène du 2-éthylhexanoate de rhodium ou de

l'acétate de rhodium.

**7.** Utilisation d'un produit de réaction à base de rhodium, dont la préparation comprend la réaction d'un ou plusieurs sels de rhodium d'un composé organique choisi dans le groupe des sels de rhodium, ce groupe étant constitué des sels de rhodium(III) de bêta-dicétones et des sels de rhodium d'acides carboxyliques, en présence de composés organophosphorés ou de leurs sels, avec du monoxyde de carbone et de l'hydrogène, pour augmenter l'activité d'un catalyseur à base de rhodium dans une opération d'hydroformylation, ledit produit de réaction étant ajouté au procédé d'hydroformylation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0544091 A
- DE 19940249 A1
- EP 0269964 A
- EP 0246475 A
- EP 0695734 B1
- US 3527809 A
- US 4148830 A
- US 4247486 A
- US 4283562 A
- DE 2627354
- EP 0103810 A
- EP 0163234 A
- EP 0571819 A
- EP 0575785 A
- EP 0640588 A